Europäisches Patentamt

**European Patent Office**

**Office européen des brevets**

(19)

(11) **EP 1 095 063 B1**

(12) **EUROPÄISCHE PATENTSCHRIFT**

(45) Veröffentlichungstag und Bekanntmachung des Hinweises auf die Patenterteilung:
**05.11.2003 Patentblatt 2003/45**

(51) Int Cl.$^7$: **C08B 30/12**, C08B 37/00, A23L 1/308

(21) Anmeldenummer: **99929219.6**

(22) Anmeldetag: **15.06.1999**

(86) Internationale Anmeldenummer:
**PCT/EP99/04129**

(87) Internationale Veröffentlichungsnummer:
**WO 00/002926 (20.01.2000 Gazette 2000/03)**

(54) **ALPHA-AMYLASE RESISTENTE POLYSACCHARIDE, VERFAHREN ZU DEREN HERSTELLUNG, VERWENDUNG, UND LEBENSMITTEL ENTHALTEND DIESE POLYSACCHARIDE**

ALPHA-AMYLASE RESISTANT POLYSACCHARIDES, PRODUCTION METHOD AND USE THEREOF AND FOOD PRODUCTS CONTAINING SAID POLYSACCHARIDES

GLUCIDES RESISTANTS A L'ALPHA-AMYLASE, LEUR PROCEDE DE FABRICATION ET LEUR UTILISATION, ET PRODUITS ALIMENTAIRES LES CONTENANT

(84) Benannte Vertragsstaaten:
**AT BE CH CY DE DK ES FI FR GB GR IE IT LI LU MC NL PT SE**

(30) Priorität: **09.07.1998 DE 19830618**

(43) Veröffentlichungstag der Anmeldung:
**02.05.2001 Patentblatt 2001/18**

(73) Patentinhaber: **Celanese Ventures GmbH**
**65926 Frankfurt am Main (DE)**

(72) Erfinder:
• **BENGS, Holger**
  **D-60598 Frankfurt am Main (DE)**
• **JACOBASCH, Gisela**
  **D-16348 Wandlitz (DE)**
• **SCHMIEDL, Detlef**
  **D-10119 Berlin (DE)**
• **RIESMEIER, Jörg**
  **D-14165 Berlin (DE)**

• **QUANZ, Martin**
  **D-10557 Berlin (DE)**
• **BÄUERLEIN, Michael**
  **D-10967 Berlin (DE)**
• **PROVART, Nicholas**
  **D-14163 Berlin (DE)**

(74) Vertreter: **Luderschmidt, Schüler & Partner Patentanwälte**
**Industriepark Höchst**
**65926 Frankfurt (DE)**

(56) Entgegenhaltungen:
**EP-A- 0 564 893          WO-A-90/15147**
**WO-A-95/31553          WO-A-98/54973**
**WO-A-99/22606**

Bemerkungen:
Die Akte enthält technische Angaben, die nach dem Eingang der Anmeldung eingereicht wurden und die nicht in dieser Patentschrift enthalten sind.

**Beschreibung**

Der Einsatz resistenter Stärken (RS) hat für die Lebensmittelindustrie eine zunehmend größere Bedeutung. Aus dem Abbau von RS-Produkten gewinnt der Organismus nur in geringem Umfang Energie. Diese Energiezufuhr bezieht sich ausschließlich auf den oxidativen Abbau resorbierter kurzkettiger Fettsäuren aus dem Dickdarm. Diese kurzkettigen Fettsäuren sind Endprodukte des Kohlenhydratstoffwechsels der intestinalen Mikroflora. Mit der Aufnahme RS-haltiger Lebensmittel sind zwei Funktionen verknüpft, Substratbereitstellung für den Energiestoffwechsel der intestinalen Mikroflora und den der Dickdarmepithelzellen. Letztere sind zur Aufrechterhaltung ihrer Struktur und Funktion auf eine luminale Zufuhr der kurzkettigen Fettsäuren und insbesondere von Butyrat angewiesen.

Seit langem ist bekannt, daß der Gehalt an hochverzweigtem Amylopektin in Stärken, die gewöhnlich aus Amylose und Amylopektin variierender Zusammensetzung bestehen, durch gezielte enzymatische Behandlung reduziert wird, wodurch der Anteil an kurzkettigen Amylosestrukturen erhöht werden kann (US Patent 3,729,380). Es ist ebenfalls bekannt, daß derartige Produkte eine stärkere Tendenz zur Retrogradation als native Stärken besitzen. Bei diesem Prozeß bilden sich sogenannte (α-amylaseresistente Stärkestrukturen aus. Als resistente Stärke (RS) werden Kohlenhydratpolymere bezeichnet, die durch (α-Amylase nicht abgebaut werden. Sie stellen dadurch eine energiereduzierte, körpergebende Komponente in Lebensmittelzusammensetzungen im Sinne eines Ballaststoffes dar. Die Behandlung mit entzweigenden Enzymen findet aus technischen Gründen gewöhnlich in einem nicht zu konzentrierten wässrigen Stärkekleister statt.

EP 0 564 893 A1 beschreibt und beansprucht einen Prozeß für die Herstellung eines RS-Produktes, das bis zu 15 % RS enthält. Dieser Prozeß ist dadurch gekennzeichnet, daß die wässrige Suspension einer Stärke, die mindestens 40 % Amylose enthält, verkleistert und enzymatisch durch Behandlung mit einem Enzym, das die α-1, 6-glykosidischen Bindungen öffnet, entzweigt und anschließend das entstandene Zwischenprodukt retrogradiert wird. Nach EP 0 564 893 A1 liegt die optimale Stärkekonzentration in der Suspension bei 15 % und die Beispiele dieser EP-Patentanmeldung illustrieren den Prozeß, wenn die Stärkekonzentrationen entweder auf 14 % reduziert bzw. auf 17 % erhöht werden. Das Ausgangsmaterial enthält mindestens 40 % Amylose und ist eine Maisstärke. Es wird weiterhin gezeigt, daß bei einem Amylosegehalt von 25 % durch diesen Prozeß keine resistente Stärke (RS) gebildet wird. Außerdem wird nachgewiesen, daß bei Erhöhung des Amylosegehaltes über 40 % auf bis zu 100 % ein Produkt erzeugbar ist, daß bis zu 50,3 % RS enthält.

EP 0 688 872 A1 beschreibt und beansprucht einen Prozeß für die Herstellung eines RS-haltigen Produktes, das 25 bis 50 Gew.-% an RS enthält. Entsprechend den Angaben beschreibt und beansprucht EP 0 688 872 A1 einen Prozeß für die Herstellung eines RS-haltigen Produktes, in dem eine wässrige Suspension einer partiell abgebauten, verkleisterten Stärke enzymatisch entzweigt und das Zwischenprodukt retrogradiert wird.

(In diesem Zusammenhang wird als "partiell abgebaute Stärke" ein Polymer verstanden, das durch geeignete Behandlung im Molekulargewicht reduziert wurde, wobei die Verkürzung der Kettenlänge sowohl die Amylose als auch das Amylopektin betrifft. Die Degradation schließt sowohl Hydrolyseprozesse (säure- oder enzymkatalysierte) als auch Extrusion, Oxidation oder Pyrolyse ein.)

Besonders hervorgehoben werden säureabgebaute Wurzel- oder Knollenstärken sowie Maltodextrine von Wurzel- oder Knollenstärken. Maltodextrine sind durch einen DE-Wert (DE: Dextrose Aquivalent) im Bereich von 1 bis 19 charakterisiert.

Sie werden aus Kartoffel- oder Tapiokastärke, die bis zu 25 % Amylose enthalten, hergestellt. Die wässrige Suspension solcher Maltodextrine enthält für den Prozeß einen Feststoffanteil von 20 Gew.-% oder mehr. Die Maltodextrine sind weiterhin dadurch charakterisiert, daß sie hohe Gehalte an Oligomeren mit Polymerisationsgraden kleiner als 10 (DP < 10) von bis zu 22 Gew.-% enthalten sowie ein mittleres Molekulargewicht von $1,3680 \times 10^4$ g/mol. Die entzweigenden Enzyme, die für den bekannten Prozeß genutzt werden, sind Pullulanase und Isoamylase. Am Ende der enzymatischen Behandlung wird eine Retrogradation in einem Temperaturbereich von 0 bis 30 °C in einem Zeitintervall von 1 bis zu 3 Tagen durchgeführt, in dem man das wässrige Reaktionsprodukt stehen läßt. Anschließend wird das Produkt durch Sprühtrocknung getrocknet. Es wird ein pulverförmiges Produkt mit einem RS-Gehalt bis zu maximal 60 Gew.-% hergestellt.

Die Erfindungsbeschreibung dient dem Ziel, Kohlenhydratpolymere mit einem hohen Anteil resistenter, relativ thermostabiler Strukturen ökonomisch herzustellen, um sie in der Lebensmittelherstellung einsetzen zu können.

So betrifft eine Ausführungsform der Erfindung α-Amylase-resistente Polysaccharide, die Poly-(1,4-α-D-glukane) sind, dadurch gekennzeichnet, daß sie einen RS-Gehalt von mindestens 65 Gew.-% aufweisen und nachdem in den Ansprüchen 1 und 9 beschriebenen Verfahren erhältlich sind.

Im Zusammenhang mit der vorliegenden Erfindung versteht man unter einem RS-Gehalt den Gehalt an α-Amylase-resistenten Polysacchariden, wie er nach der Methode von Englyst et al. (Classification and measurement of nutritionally important starch Fractions, European Journal of Clinical Nutrition, 46 (Suppl. 23) (1992) 33-50) bestimmt werden kann; s. auch Beispiel 3.

Die erfindungsgemäßen α-Amylase-resistenten Polysaccharide können gekennzeichnet sein durch einen RS-Gehalt von mindestens 75 und insbesondere mindestens 95 Gew.-%.

Ferner können die erfindungsgemäßen $\alpha$-Amylase-resistenten Polysaccharide dadurch gekennzeichnet sein, daß die Poly-(1,4-$\alpha$-D-glukane) chemisch in an sich bekannter Weise modifiziert sind.

So können die Poly- (1,4-$\alpha$-D-glukane) durch Veretherung oder Veresterung in 2-, 3- oder 6-Position chemisch modifiziert worden sein. Der Fachmann ist mit chemischer Modifizierung hinlänglich vertraut; vgl. beispielsweise folgende Literatur:

1. Functional Properties of Food Components, 2nd edition, Y. Pomeranz, Academic Press (1991).
2. Lehrbuch der Lebensmittelchemie, Belitz & Grosch, Springer Verlag (1992).
3. Citrat Starch Possible Application as Resistent Starch in Different Food Systems, B. Wepner et al., European Air Concerted Action, Abstract: air3ct94-2203, Functional Properties of Non-digestible Carbohydrates, Pro Fibre-Tagung, Lissabon, Februar 1998, Seite 59.

Ferner können die erfindungsgemäßen $\alpha$-Amylase-resistenten Polysaccharide dadurch gekennzeichnet sein, daß sie in 6-Position einen Verzweigungsgrad von höchstens 0,5 % aufweisen.

Ferner können die erfindungsgemäßen $\alpha$-Amylase-resistenten Polysaccharide dadurch gekennzeichnet sein, daß sie in 2- und/oder 3-Position einen Verzweigungsgrad von jeweils höchstens 1,0 % und insbesondere höchstens 0,5 % aufweisen.

Ferner können die $\alpha$-Amylase-resistenten Polysaccharide dadurch gekennzeichnet sein, daß die Poly-(1,4-$\alpha$-D-glukane) ein Molekulargewicht von 0,75 x $10^2$ bis $10^7$, bevorzugt $10^3$ bis $10^6$ und bevorzugt von $10^3$ bis 5 x $10^5$ g/mol aufweisen und/oder wasserunlöslich sind.

Ferner können die erfindungsgemäßen $\alpha$-Amylase-resistenten Polysaccharide dadurch gekennzeichnet sein, daß die Poly-(1,4-$\alpha$-D-glukane) weder entzweigt, insbesondere weder enzymatisch entzweigt, noch hinsichtlich ihrer Kettenlänge (und damit hinsichtlich ihres Molekulargewichtes) reduziert worden sind, insbesondere nicht durch Enzymkatalyse, Säurekatalyse, Extrusion, Oxidation oder Pyrolyse.

Ferner können die erfindungsgemäßen $\alpha$-Amylase-resistenten Polysaccharide nach einem Verfahren erhältlich sein umfassend folgende Schritte:

a) Bereitstellung von wasserlöslichen Poly-(1,4-$\alpha$-D-glukan) durch die Umsetzung von Saccharose mit einem Amylosucrase-Aktivität aufweisendem Enzym;
b) Herstellung einer Suspension oder Dispersion aus wasserunlöslichen Poly-(1,4-$\alpha$-D-glukanen) und Wasser;
c) Erwärmung der Suspension oder Dispersion;
d) Abkühlung des erhaltenen Kleisters und Retrogradation des Kleisters bei einer gegenüber der Temperatur des erhitzten Kleisters erniedrigten Temperatur; und
e) ggf. Trocknung des erhaltenen Produktes.

Unter dem Begriff "wasserunlöslich" versteht man Verbindungen, die nach der Definition des Deutschen Arzneimittelbuches (Wissenschaftliche Verlagsgesellschaft/Stuttgart & Gori-Verlag/Frankfurt, 9. Aufl., 1987; s. auch Beispiele 22 bis 23) unter die Kategorie "schwer lösliche" Verbindungen, "sehr schwer lösliche" oder "praktisch unlösliche" Verbindungen fallen.

Der Fachmann ist mit den Begriffen "Suspension" und "Dispersion" vertraut. Ergänzend sei verwiesen auf Römpp, Chemie-Lexikon, 9. Auflage, Thieme-Verlag, Stuttgart & New York, Seiten 4401 bzw. 1010.

Der Fachmann ist ferner mit dem Begriff "Kleister" vertraut. Ergänzend sei verwiesen auf Römpp, Chemie-Lexikon, 9. Auflage, Thieme-Verlag, Stuttgart & New York, Seite 2256.

Ferner können die erfindungsgemäßen $\alpha$-Amylase-resistenten Polysaccharide nach einem Verfahren erhältlich sein umfassend folgende Schritte:

a) Bereitstellung von wasserunlöslichem Poly-(1,4-$\alpha$-D-glukan) durch die Umsetzung von Saccharose mit einem Amylosucrase-Aktivität aufweisenden Enzym,
b) Herstellung einer Suspension oder Dispersion aus wasserunlöslichen Poly-(1,4-$\alpha$-D-glukanen) und Wasser;
c) Einfrieren der erhaltenen Suspension oder Dispersion;
d) Retrogradation;
e) Auftauen der nach Schritt c) erhaltenen Masse; und
f) ggf. Trocknung der nach Schritt d) erhaltenen Masse oder Entwässerung der erhaltenen Masse.

Eine weitere Ausführungsform der Erfindung betrifft ein Verfahren zur Herstellung von $\alpha$-Amylase-resistenten Polysacchariden mit hohem RS-Gehalt umfassend folgende Schritte:

a) Bereitstellung von wasserunlöslichem Poly-(1,4-α-D-glukan) durch die Umsetzung von Saccharose mit einem Amylosucrase-Aktivität aufweisenden Enzym,

b) Herstellung einer Suspension oder Dispersion aus wasserunlöslichen Poly-(1,4-α-D-glukanen) und Wasser;

c) Erwärmung der Suspension oder Dispersion;

d) Abkühlung des erhaltenen Kleisters und Retrogradation des Kleisters bei einer gegenüber der Temperatur des erhitzten Kleisters erniedrigten Temperatur; und

d) ggf. Trocknung des erhaltenen Produktes.

Der Vorteil des erfindungsgemäßen Verfahrens kann darin bestehen, daß aus den oben beschrieben Ausgangsstoffen ein wässriger heißer Kleister herstellbar ist, der bis zu beispielsweise 30 Gew.-% oder mehr Feststoffanteile enthält, ohne daß beispielsweise eine Entzweigung oder ein partieller Abbau bzw. eine Kettenlängenreduktion der verwendeten Ausgangsstoffe vorgenommen werden muß. Dies führt zu einer Vereinfachung der Prozeßführung und damit zu einer Reduzierung der Kosten des Verfahrens, weil der zeit- und kostenaufwendige Einsatz entzweigender Enzyme bzw. degradierender Chemikalien entfällt.

Das erfindungsgemäße Verfahren kann dadurch gekennzeichnet sein, daß man bei Schritt (b) einen Kleister mit einem Polysaccharid-Gehalt von mindestens 5 und bis zu 30, 35, 40, 45 oder 50 Gew.-% herstellt.

Ferner kann das erfindungsgemäße Verfahren dadurch gekennzeichnet sein, daß man bei Schritt (c) den Kleister auf eine Temperatur im Bereich von Raumtemperatur, 50, 60 oder 70 bis 100 °C erwärmt oder erhitzt.

Ferner kann das erfindungsgemäße Verfahren dadurch gekennzeichnet sein, daß man bei Schritt (d)

(i) bei einer Temperatur im Bereich von 50 °C bis an den Gefrierpunkt, vorzugsweise 35 bis 15 °C, 27 bis 22 °C, 16 bis 0 °C oder 6 bis 2 °C und/oder

(ii) für ein Zeitintervall von 1 bis 72 h, vorzugsweise 1 bis 36 h und insbesondere 15 bis 30 h

retrogradiert.

Ferner kann das erfindungsgemäße Verfahren dadurch gekennzeichnet sein, daß man bei Schritt (d) nach einem Temperaturstufenprogramm

(i) in einem Temperaturbereich von 100 bis 0 °C und vorzugsweise 90 bis 4 °C

(ii) für ein Gesamtzeitintervall von 8 bis 36 h, vorzugsweise 20 bis 28 h und insbesondere 22 bis 26 h gemäß dem folgenden Temperatur-Zeit-Programm stufenweise und gegebenenfalls unter Einwirkung von Scherkräften abkühlt und retrogradiert, wobei sich die gewählten Zeitintervalle zu einem vorstehend angegebenen Gesamt-Zeitintervall ergänzen:

| Temperatur-Zeit-Programm | |
| --- | --- |
| Temperatur (°C) | Zeitintervall |
| 90 ± 10 | 5 min ± 5 min |
| 80 ± 10 | 10min ± 10min |
| 70 ± 10 | (30 bis 180 min) ± 30 |
| 40 ± 10 | (60 bis 180 min) ± 60 min |
| 25 ± 10 | 22 h ± 15 h |
| 4 ± 10 | 20 h ± 15 h |

Eine weitere Ausführungsform der Erfindung betrifft ein Verfahren zur Herstellung von α-Amylase-resistenten Polysacchariden mit hohem RS-Gehalt umfassend folgende Schritte:

a) Bereitstellung von wasserunlöslichem Poly-(1,4-α-D-glukan) durch die Umsetzung von Saccharose mit einem Amylosucrase-Aktivität aufweisenden Enzym,

b) Herstellung einer Suspension oder Dispersion aus wasserunlöslichen Poly-(1,4-α-D-glukanen) und Wasser;

c) Einfrieren der erhaltenen Suspension oder Dispersion;

d) Retrogradation;

e) Auftauen, der nach Schritt d) erhaltenen Masse; und

f) ggf. Trocknung der nach Schritt e) erhaftenen Masse oder Entwässerung der erhaltenen Masse.

Das erfindungsgemäße Verfahren kann dadurch gekennzeichnet sein, daß man die gemäß Schritt (c) aufgetaute Masse noch einmal oder mehrmals den Schritten (e) bis (e) unterwirft, bevor man endgültig auftaut, trocknet oder entwässert.

Ferner kann das erfindungsgemäße Verfahren dadurch gekennzeichnet sein, daß man bei Schritt (c) die erhaltene Suspension oder Dispersion auf eine Temperatur im Bereich 0 °C bis -80 °C abkühlt.

Ferner kann das erfindungsgemäße Verfahren dadurch gekennzeichnet sein, daß man bei Schritt (d) für ein Zeitintervall von 1 bis 72 h, bevorzugt 1 bis 36 h und insbesondere 15 bis 30 h retrogradiert.

Die Ausführungsformen des erfindungsgemäßen Verfahrens ist dadurch gekennzeichnet, daß man von Poly-(1,4-$\alpha$-D-glukanen) ausgeht, die aus Biotransformation, aus Umsetzung von Saccharose mit einem Enzym der enzymatischen Aktivität einer Amylosucrase gewonnen worden sind; vgl. beispielsweise WO 95 31 553.

Unter einer Amylosucrase versteht man ein Enzym, das die folgende Reaktion katalysiert:

$$\text{Saccharose} + (\alpha\text{-1,4-Glukan})_n \leftrightarrow \text{Fruktose} + (\alpha\text{-1,4-Glukan})_{n+1}$$

Ausgehend von diesem Reaktionsschema können lineare oligomere oder polymere $\alpha$-1,4-Glukane als Akzeptoren für eine kettenverlängernde Reaktion dienen, die zu wasserunlöslichen Poly-(1,4-$\alpha$-D-glukanen) führt, deren Glukoserest durch $\alpha$-1,4-glykosidische Bindungen verknüpft sind und die ein Molekulargewicht im Bereich von 0,75 x $10^2$ g/mol bis $10^7$ g/mol aufweisen.

Die linearen oligomeren oder polymeren Akzeptoren können dabei entweder von außen zugesetzt werden, sie können jedoch auch, wie in Beispiel 1 beschrieben, durch die Amylosucrase selbst aus Saccharose erzeugt werden.

$\alpha$-1,6-glykosidische Bindungen sind in diesen Produkten per $^{13}$C-NMR nicht nachweisbar (Remaud-Simeon et al. in Carbohydrate Bioengeneering (ed. S. B. Petersen et al.), Elsevier Science B.V. (1995), 313-320).

In einer weiteren bevorzugten Ausführungsform des erfindungsgemäßen Verfahrens verwendet man wasserunlösliche Poly-(1,4-$\alpha$-D-glukane) als Ausgangsstoffe, die durch die Umsetzung von Saccharose mit einem Enzym mit der enzymatischen Aktivität einer Amylosucrase unter Einsatz von verzweigten Polysaccharidakzeptoren, wie z. B. Glykogen, Amylopektin, Dextrin, gewonnen werden können. Die Amylosucrase katalysiert eine $\alpha$-1,4-Glukankettenverlängerung an diesen verzweigten Polysaccharidakzeptoren. Die dabei entstehenden wasserunlöslichen Poly-(1,4-$\alpha$-D-glukane) weisen im Vergleich zu den eingesetzten verzweigten Polysaccharidakzeptoren einen geringeren Verzweigungsgrad auf. Auch diese Produkte werden im Rahmen der vorliegenden Erfindung als Poly-(1,4-$\alpha$-D-glukane) bezeichnet, die die eben beschriebenen Eigenschaften aufweisen, aber auf anderem Wege erzeugt worden sind, können Ausgangsstoffe des erfindungsgemäßen Verfahrens sein.

Ferner können die Ausführungsformen des erfindungsgemäßen Verfahrens dadurch gekennzeichnet sein, daß die wasserunlöslichen Poly-(1,4-$\alpha$-D-glukane) chemisch in an sich bekannter Weise modifiziert sind.

Ferner können die Ausführungsformen des erfindungsgemäßen Verfahrens dadurch gekennzeichnet sein, daß die wasserunlöslichen Poly-(1,4-$\alpha$-D-glukane) in 6-Position einen Verzweigungsgrad von höchstens 0,5 % aufweisen.

Ferner können die Ausführungsformen des erfindungsgemäßen Verfahrens dadurch gekennzeichnet sein, daß die wasserunlöslichen Poly-(1,4-$\alpha$-D-glukane) in 2- oder 3-Position einen Verzweigungsgrad von jeweils höchstens 1 % und insbesondere höchstens 0,5 % aufweisen.

Ferner können die Ausführungsformen des erfindungsgemäßen Verfahrens dadurch gekennzeichnet sein, daß die wasserunlöslichen Poly-(1,4-$\alpha$-D-glukane) ein Molekulargewicht von 0,75 x $10^2$ bis $10^7$, bevorzugt $10^3$ bis $10^6$ g/mol und bevorzugt von $10^3$ bis 5 x $10^5$ aufweisen.

Ferner können die Ausführungsformen des erfindungsgemäßen Verfahrens dadurch gekennzeichnet sein, daß die wasserunlöslichen Poly-(1,4-$\alpha$-D-glukane) weder entzweigt, insbesondere weder enzymatisch entzweigt, noch hinsichtlich ihrer Kettenlänge (und damit hinsichtlich ihres Molekulargewichts) reduziert worden sind, insbesondere nicht durch Enzymkatalyse, Säurekatalyse, Extrusion, Oxidation oder Pyrolyse.

Unter dem Begriff "hoher RS-Gehalt" versteht man einen RS-Gehalt von mindestens 65 bis 75, 75 bis 88, 88 bis 90, 90 bis 95 und insbesondere 95 bis 99 oder mehr Gew.-%.

So sind die Ausführungsformen des erfindungsgemäßen Verfahrens dadurch gekennzeichnet, daß man $\alpha$-Amylase-resistente Polysaccharide mit einem RS-Gehalt von mindestens 65 Gew.-% gewinnt.

Ferner können die Ausführungsformen des erfindungsgemäßen Verfahrens dadurch gekennzeichnet sein, daß man bei den Trocknungsschritten (f) bzw. (e) das retrogradierte Produkt durch Sprüh- oder Gefriertrocknung trocknet. Eine weitere Ausführungsform der Erfindung betrifft eine Verwendung eines erfindungsgemäßen $\alpha$-Amylase-resistenten Polysaccharids für Lebensmittel-Vorprodukte oder Lebensmittel.

Eine weitere Ausführungsform der Erfindung betrifft eine Verwendung eines erfindungsgemäßen $\alpha$-Amylase-resistenten Polysaccharids als Lebensmitteladditiv oder Lebensmittelzusatzstoff.

Schließlich betrifft eine Ausführungsform der Erfindung ein Lebensmittel-Vorprodukt oder Lebensmittel, gekennzeichnet durch einen Gehalt an einem erfindungsgemäßen $\alpha$-Amylase-resistenten Polysaccharid.

Die folgenden Beispiele dienen zur näheren Erläuterung der Erfindung, ohne diese auf die Beispiele zu begrenzen.

Beispiel 1: Biotransformation

In einem 5-l-Gefäß werden 5 l einer sterilisierten 30-proz. Saccharose Lösung gegeben. Ein Enzymextrakt, der eine Amylosucrase aus Neisseria polysaccharea enthält (s. WO 95 31 553), wird in einer Portion zugegeben und gemischt. Die eingesetzte Enzymaktivität beträgt in diesem Experiment 148000 Units. Das verschlossene Gefäß wurde bei 37 °C inkubiert. Während der Dauer der Biotransformation bildet sich ein weißer Niederschlag. Die Reaktion wird nach 39 h beendet. Der Niederschlag wird abzentrifugiert, bei -70 °C eingefroren und anschließend gefriergetrocknet. Die Masse des gefriergetrockneten Feststoffes beträgt 526,7 g (70,2 % Ausbeute).

Zur Abtrennung niedermolekularer Zucker werden 200 g des Feststoffes mit Wasser 30 min unter Rühren bei Raumtemperatur gewaschen, bei -70 °C eingefroren und gefriergetrocknet. Der Gehalt an Fruktose und Saccharose wird nach Lösen des Feststoffes in DMSO durch einen gekoppelten enzymatischen Assay[1] bestimmt und beträgt 4,61 mg Fruktose pro 100 mg Feststoff (4, 6 %). Der Gehalt an Saccharose liegt unter der Nachweisgrenze.

Der Überstand der Biotransformation wird bei 95 °C denaturiert. Nach Abkühlen auf Raumtemperatur wurde erneut zentrifugiert. Der klare Überstand wurde bei -70 °C eingefroren und über 3 Tage bei 4 °C aufgetaut. Der so erzeugte Niederschlag wurde bei -70 °C eingefroren und gefriergetrocknet.

Zur Abtrennung niedermolekularer Zucker werden 39, 5 g des Feststoffes mit Wasser 30 min unter Rühren bei Raumtemperatur gewaschen, bei -70 °C eingefroren und gefriergetrocknet. Der Gehalt an Fruktose und Saccharose wird nach Lösen des Feststoffes in DMSO durch einen gekoppelten enzymatischen Assay gemäß STITT et al. (Meth. Enzym., 174 (1989) 518 - 552) bestimmt und beträgt 2,27 mg Fruktose pro 100 mg Feststoff. Der Gehalt an Saccharose liegt unter der Nachweisgrenze.

Beispiel 2: Ausgangsmaterial

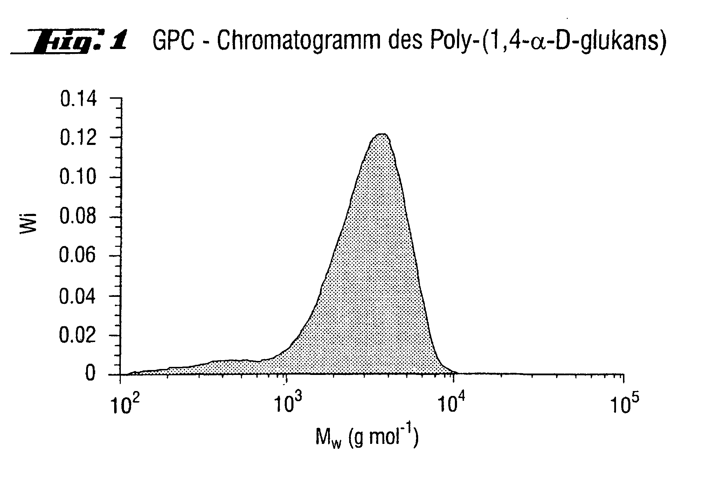

Bestimmung des Molekulargewichts des mit Amylosucrase synthetisierten wasserunlöslichen Poly-(1,4-$\alpha$-D-glukans) aus Beispiel 1 (Figur 1)

Es werden 2 mg des Poly-(1,4-$\alpha$-D-glukans) aus Beispiel 1 bei Raumtemperatur in Dimethylsulfoxid (DMSO, p.a. von Riedel-de-Haen) gelöst und filtriert (2 $\mu$m). Ein Teil der Lösung wird in eine Säule der Gelpermeationschromatographie infiziert. Als Elutionsmittel wird DMSO verwendet. Die Signalintensität wird mittels eines RI-Detektors gemessen und gegen Pullulanstandards (Firma Polymer Standard Systems) ausgewertet. Die Flußrate beträgt 1.0 ml pro Minute.

Die Messung ergibt ein Zahlenmittel des Molekulargewichts ($M_n$) von 2.326 g/mol und ein Gewichtsmittel des Molekulargewichts ($M_w$) von 3.367 g/mol. Die Wiederfindungsrate beträgt 100 %.

Beispiel 3

Beispiel zur Bestimmung des RS-Gehaltes.

200 mg (Trockengewicht) eines auf seinen RS-Gehalt zu analysierenden pulverförmigen Produktes wurden nach der Methode von Englyst et al. (Eur. J. Clin. Nutrition, 46 (1992) (Suppl. 2) S 33-550) zur Bestimmung des RS-Gehaltes mit der beschriebenen Enzymmischung bei pH 5,2 120 min inkubiert. Nach Beendigung des enzymatischen Abbaus wurde die Aktivität der Enzyme durch Erniedrigung des pH-Wertes auf einen Wert von 3 und der Temperatur auf 20 °C gestoppt. Anschließend erfolgte durch Zugabe der 4-fachen Menge an Ethanol die Einstellung einer 80-proz. (v/v) ethanolischen Lösung. Die 80-proz. ethanolische Lösung wurde für 1 h bei Raumtemperatur stehengelassen. Das Präzipitat wurde zentrifugiert (2500 x g, 10 min) und der Überstand verworfen. Der Rückstand wurde dreimal mit 80-proz. (v/v) Ethanol und einmal mit absolutem Ethanol gewaschen und zentrifugiert. Der Rückstand wurde lyophilisiert und gewogen. Die Trockenmasse des Rückstandes wurde bestimmt und der RS-Gehalt nach folgender Gleichung berechnet:

RS [%] = 100 x Gewicht des Rückstandes (Trockengewicht)/Einwaage

(Trockengewicht)

Beispiele 4 bis 7

Ein lineares, naturidentisches Poly-(1,4-α-D-glukan) (siehe Beispiel 1) wurde in wässriger Lösung erhitzt und ein Kleister gebildet. Dieser Kleister wurde auf 10 Gew.-% Feststoffanteil eingestellt und portioniert. Die Portionen wurden bei 4 und 25 °C (Beispiel 5 bzw. 6) oder mit Hilfe eines Stufenprogramms (Beispiel 7) retrogradiert. Des weiteren wurde das lineare Kohlenhydratpolymer aus dem Reaktionsansatz ausgefroren (Beispiel 4). Die retrogradierten Muster wurden getrocknet und die Bestimmung des RS-Gehaltes wie oben beschrieben durchgeführt.

Tabelle 2 illustriert den Einfluß der Retrogradationstemperatur und -bedingungen auf den RS-Gehalt im Produkt, hergestellt aus einem 10-proz. Kleister der verwendeten Poly-(1,4-α-D-glukane) durch 24-stündige Retrogradation.

Tabelle 2

| Beispiel | Retrogradationstemperatur | RS [Gewicht-%] |
|---|---|---|
| 4 | -70 °C | 78 ± 4 |
| 5 | 4 °C | 70 ± 2 |
| 6 | 25 °C | 87 ± 1 |
| 7 | Stufenprogramm | 74 ± 3 |

Dieses Beispiel in Tabelle 2 zeigt, daß die Retrogradationstemperatur den RS-Gehalt beeinflußt. So führt eine Retrogradation bei 25 °C zu einem deutlich höheren RS-Anteil verglichen mit einer Retrogradation bei 4 °C. Durch Retrogradation bei-70 °C erhält man hingegen einen leicht höheren RS-Anteil verglichen mit dem nach Retrogradation bei 4 °C. Das Ausgangsprodukt verhält sich dementsprechend anders als Maltodextrine, wie sie in EP 0 688 872 A1 beschrieben und beansprucht werden.

Beispiele 8 bis 12

Das gleiche wie unter Beispielen 4 bis 7 verwendete Poly-(1,4-α-D-glukan) wurde in wässriger Lösung erhitzt, um einen Kleister zu bilden. Dieser wurde auf 10 und 30 Gew.-% Feststoffanteil eingestellt und portioniert. Die Portionen wurden bei 4 und 25 °C oder mit Hilfe eines Stufenprogramms retrogradiert. Tabelle 3 illustriert den Einfluß des Feststoffanteils im Kleister auf den RS-Gehalt im Produkt, hergestellt aus 10 und 30-proz. Kleister des verwendeten Poly-(1,4-α-D-glukans) durch 24-stündige Retrogradation.

Tabelle 3

| Beispiel | Retrogradationstemperatur | Feststoffanteil | |
|---|---|---|---|
| | | 10 % RS [Gewicht-%] | 30 % RS [Gewicht-%] |
| 8 | 4 °C | 70 ± 2 | |
| 9 | 4 °C | | 94 ± 2 |
| 10 | 25 °C | 87 ± 1 | |
| 11 | 25 °C | | 93 ± 1 |
| 12 | Stufenprogramm | 74 ± 3 | |

Dieses Beispiel in Tabelle 3 zeigt, daß der Feststoffanteil im Kleister den RS-Gehalt beeinflußt. So führt eine Retrogradation bei 30 % Feststoffanteil zu einem deutlich höheren RS-Gehalt im Produkt verglichen mit einer Retrogradation bei einem 10-proz. Feststoffanteil. Das Ausgangsprodukt verhält sich dementsprechend anders als Maltodextrine, wie sie in EP 0 688 872 A1 beschrieben und beansprucht werden.

Beispiele 13 bis 21

Das gleiche wie unter Beispielen 4 bis 7 verwendete Poly-(1,4-α-D-glukan) wurde in wässriger Lösung erhitzt, um einen Kleister zu bilden. Dieser wurde auf 10 bzw. 30 Gew.-% Feststoffanteil eingestellt und portioniert. Die Portionen wurden bei -70, 4 und 25 °C retrogradiert. Danach wurden die erhaltenen Produkte getrocknet und die thermische Stabilität mit Hilfe der dynamischen Differenzkaloriemetrie (DSC) untersucht.

Die DSC-Messung liefert für die Quellung von nativer Stärke einen endothermen Peak. Ähnliches trifft auch für retrogradierte Stärken und Poly-(1,4-α-D-glukane) zu. An Hand von Endothermen lassen sich die Vorgänge des Schmelzens der Kristallite, der Konformationsänderung sowie der Hydratation und Quellung von Stärkepolymeren charakterisieren.

Messungen unter der Bedingung des Wasserüberschusses (Wassergehalte über 60 %) ergeben in der Regel einen einheitlichen Peak. Dieser Peak ist durch verschiedene Parameter, wie on set-Temperatur $T_0$, Peaktemperatur $T_p$, Endtemperatur $T_c$, sowie Reaktionsenthalpie dH (Fläche des Peaks) charakterisiert. Die o. g. Bedingung ist für alle in Tabelle 4 aufgeführten Parameter erfüllt.

Die Messungen wurden mit einem hochauflösenden Gerät (DSC120, Fa. Seiko, Japan) durchgeführt. Das Glukan/Wasser-Verhältnis betrug 1:5, die Heizrate 4 K/min. Die Messung wurde in einem Temperaturbereich von 10 bis 220 °C durchgeführt. Das Gerät arbeitet nach dem Heat Flux-Meßprinzip. Pro Messung wurden 5 mg Polyglukan mittels Ultra-Mikrowaage in Silbertiegel von 70 µl Fassungsvermögen eingewogen und diese nach Zugabe von destilliertem Wasser anschließend hermetisch verschlossen. Als Referenzprobe wurde destilliertes Wasser mit einer Leitfähiqkeit von 0,15 µS verwendet,

Tabelle 4

| Bezeichnung Poly-(1,4-α-D-glukan) | Retrogradations-bedingungen Temperatur/Feststoffanteil im Kleister | DSC-Parameter | | | |
|---|---|---|---|---|---|
| | | $T_0$ [°C] | $T_p$ [°C] | $T_c$ [°C] | dH [J/g] |
| Beispiel | | | | | |
| 13 | Tieftemperatur-rekristallisation | 85,1 | 102,0 | 111,3 | 21,8 |
| 14 | 4 °C, 10 % | 81,8 | 96,7 | 108,0 | 16,3 |
| 15 | 4 °C, 30 % | 86,2 | 98,2 | 103,7 | 1,8 |
| 16 | | 109,3 | 124,5 | 136,8 | 13,3 |
| 17 | | 142,7 | 154,1 | 165,6 | 2,9 |
| 18 | 25 °C, 10 % | 88,6 | 101,0 | 109,8 | 15,0 |
| 19 | 25 °C, 30 % | 85,9 | 97,9 | 101,1 | 1,7 |
| 20 | | 111,8 | 126,1 | 133,9 | 4,0 |
| 20 | | 138,1 | 157,6 | 172,5 | 23,0 |

Diese Beispiele in Tabelle 4 zeigen, daß der Feststoffanteil im Kleister die thermische Stabilität der retrogradierten Produkte beeinflußt. So führt eine Retrogradation bei 30 % Feststoffanteil im Kleister zu Produkten, die in DSC-Messungen Endothermen mit mehr als einem Peak zeigen, wobei Peaktemperaturen ($T_p$) in diesen Endothermen von > 120 °C auftreten. Hingegen führt die Retrogradation 10-proz. Kleister zu Produkten, deren Endothermen nur einen Peak mit $T_p$-Werten zwischen 95 und 100 °C aufweisen. Die Erhöhung des Feststoffanteils im Kleister bewirkt somit eine Erhöhung der thermischen Stabilität des retrogradierten Produktes.

Beispiel 22

Bestimmung der Löslichkeit von Polysacchariden und Klassifizierung nach Deutschem Arzneimittelbuch (DAB)

564 mg Poly-(1,4-α-glukan) (siehe Beispiel 1) werden in ca. 0,5 l bidestilliertem Wasser bei 1,3 bar und 130 °C für 1,5 Stunden in einem Autoklaven erhitzt (Apparat Certoclav). Von dem Reaktionsgefäß ist zuvor das Gewicht gemessen worden. Danach wird die Apparatur entspannt und bei Raumtemperatur abgekühlt. Der Inhalt wird gewogen. Er entspricht 501,74 g. Nach weiteren 24 Stunden wird zentrifugiert und dekantiert. Der feste Rückstand wird getrocknet und ausgewogen. Es sind 468 mg. Daraus errechnet sich ein gelöster Anteil von 96 mg. Bezogen auf das eingesetzte Lösungsmittel errechnet sich daraus, daß für 1 mg Poly-(1,4-α-glukan) 5226 mg Wasser notwendig sind. Gemäß der Klassifizierung nach Deutschem Arzneimittelbuch ergibt sich daraus die Einteilung, daß diese Substanz "sehr schwer löslich" ist, da zwischen 1.000 und 10.000 Teilen Lösungsmittel notwendig sind, um 1 Teil der Substanz in Lösung zu bringen. Dies ist von den 7 Klassen zur Einteilung der Löslichkeit (von "sehr leicht löslich" (Klasse 1) bis "praktisch unlöslich" (Klasse 7) die Klasse Nummer 6.

Beispiel 23

Bestimmung der Löslichkeit von Polysacchariden und Klassifizierung nach Deutschem Arzneimittelbuch (DAB)

Der Versuch wird wie in Beispiel 22 durchgeführt. Der einzige Unterschied bildet ein Kühlprozeß, der nach der Autoklavbehandlung und dem Abkühlen auf Raumtemperatur nachgeschaltet wird. Das Substanzgemisch wird für 3 Stunden bei 5 °C aufbewahrt.

Es werden 526 mg Poly-(1,4-α-glukan) auf ca. 480 ml bidestilliertem Wasser eingewogen. Nach der thermischen Behandlung ergibt sich eine Auswaage von 468,09 g. Das getrocknete Sediment beträgt 488 mg. Demnach sind 38 mg des Poly-(1,4-α-glukans) in Lösung gegangen. Dies entspricht einem Verhältnis von 1 mg Substanz zu 12.305 Teilen Lösungsmittel. Demnach ist die Substanz nach dieser Behandlungsmethode in Klasse Nummer 7 nach DA.B einzustufen und danach als praktisch unlöslich zu klassifizieren, weil mehr als 10.000 Teile Lösungsmittel für ein Teil Substanz benötigt werden.

## Patentansprüche

1. α-Amylase-resistente Polysaccharide, **dadurch gekennzeichnet, dass** sie einen nach der Methode von Englyst et al. ermittelten RS-Gehalt von mindestens 65 Gew.-% aufweisen, erhältlich nach einem Verfahren, das folgende Schritte umfasst:

   a) Bereitstellung von wasserunlöslichem Poly-(1,4-α-D-glukan) durch die Umsetzung von Saccharose mit einem Amylosucrase-Aktivität aufweisenden Enzym,
   b) Herstellung einer Suspension oder Dispersion aus dem Poly-(1,4-α-D-glukan) und Wasser,
   c) Erwärmung der Suspension oder Dispersion, um einen Kleister zu erhalten;
   d) Abkühlung des erhaltenen Kleisters und Retrogradation des Kleisters bei einer gegenüber der Temperatur des erhitzten Kleisters erniedrigten Temperatur; und
   e) ggf. Trocknung oder Entwässerung des erhaltenen Produktes.

2. α-Amylase-resistente Polysaccharide nach Anspruch 1, **gekennzeichnet durch** einen RS-Gehalt von mindestens 75%.

3. α-Amylase-resistente Polysaccharide nach Anspruch 1 oder 2, **dadurch gekennzeichnet, dass** die Poly-(1,4-α-D-glukane) chemisch in an sich bekannter Weise modifiziert sind.

4. α-Amylase-resistente Polysaccharide nach einem der vorhergehenden Ansprüche, **dadurch gekennzeichnet, dass** sie in 6-Position einen Verzweigungsgrad von höchstens 0,5 % aufweisen.

5. α-Amylase-resistente Polysaccharide nach einem der vorhergehenden Ansprüche, **dadurch gekennzeichnet, dass** sie in 2- und/oder 3-Position einen Verzweigungsgrad von jeweils höchstens 0,5 % aufweisen.

6. α-Amylase-resistente Polysaccharide nach einem der vorhergehenden Ansprüche, **dadurch gekennzeichnet, dass** die Poly-(1,4-α-D-glukane) ein Molekulargewicht von 0,75 x $10^2$ bis $10^7$ g/mol aufweisen.

7. α-Amylase-resistente Polysaccharide nach einem der vorhergehenden Ansprüche, **dadurch gekennzeichnet, dass** die Poly-(1,4-α-D-glukane) ein Molekulargewicht von $10^3$ bis 5 x $10^5$ g/mol aufweisen.

8. α-Amylase-resistente Polysaccharide nach einem der vorhergehenden Ansprüche, **dadurch gekennzeichnet, dass** die Poly(1,4-α-D-glukane) weder entzweigt, insbesondere weder enzymatisch entzweigt, noch hinsichtlich ihrer Kettenlänge (und damit hinsichtlich ihres Molekulargewichtes) reduziert worden sind.

9. α-Amylase-resistente Polysaccharide, **dadurch gekennzeichnet, dass** sie einen nach der Methode von Englyst et al. ermittelten RS-Gehalt von mindestens 65 Gew.-% aufweisen, erhältlich nach einem Verfahren, das folgende Schritte umfasst:

   a) Bereitstellung von wasserunlöslichem Poly-(1,4-α-D-glukan) durch die Umsetzung von Saccharose mit einem Amylosucrase-Aktivität aufweisenden Enzym,
   b) Herstellung einer Suspension oder Dispersion aus dem Poly-(1,4-α-Dglukan) und Wasser

c) Einfrieren der erhaltenen Suspension oder Dispersion;

d) Retrogradation;

e) Auftauen der nach Schritt d) erhaltenen Masse; und

f) ggf. Trocknung oder Entwässerung der nach Schritt d) erhaltenen Masse.

**10.** Verfahren zur Herstellung von $\alpha$-Amylase-resistenten Polysacchariden, die einen nach der Methode von Englyst et al. ermittelten RS-Gehalt von mindestens 65 Gew.-% aufweisen, umfassend folgende Schritte:

a) Bereitstellung von wasserunlöslichem Poly-(1,4-$\alpha$-D-glukan) durch die Umsetzung von Saccharose mit einem Amylosucrase-Aktivität aufweisenden Enzym,

b) Herstellung einer Suspension oder Dispersion aus wasserunlöslichen Poly-(1,4-$\alpha$-D-glukanen) und Wasser;

c) Erwärmung der Suspension oder Dispersion;

d) Abkühlung des erhaltenen Kleisters und Retrogradation des Kleisters bei einer gegenüber der Temperatur des erhitzten Kleisters erniedrigten Temperatur; und

e) ggf. Trocknung oder Entwässerung des erhaltenen Produktes.

**11.** Verfahren nach Anspruch 10, **dadurch gekennzeichnet, dass** man bei Schritt (b) gemäß Anspruch 10 einen Kleister mit einem Polysaccharid-Gehalt von mindestens 5 und bis 50 Gew.-% herstellt.

**12.** Verfahren nach einem der Ansprüche 10 oder 11, **dadurch gekennzeichnet, dass** man bei Schritt (c) gemäß Anspruch 10 den Kleister auf eine Temperatur im Bereich von Raumtemperatur bis 100 °C erwärmt oder erhitzt.

**13.** Verfahren nach einem der Ansprüche 10 bis 12, **dadurch gekennzeichnet, dass** man bei Schritt (d) gemäß Anspruch 10

(i) bei einer Temperatur im Bereich von 50 °C bis an den Gefrierpunkt, und/oder

(ii) für ein Zeitintervall von 1 bis 72 h

retrogradiert.

**14.** Verfahren nach einem der Ansprüche 10 bis 13, **dadurch gekennzeichnet, dass** man bei Schritt (d) gemäß Anspruch 10 nach einem Temperaturstufenprogramm

(i) in einem Temperaturbereich von 100 bis 0 °C

(ii) für ein Gesamtzeitintervall von 8 bis 36 h gemäß dem folgenden Temperatur-Zeit-Programm stufenweise und gegebenenfalls unter Einwirkung von Scherkräften abkühlt und retrogradiert, wobei sich die gewählten Zeitintervalle zu einem vorstehend angegebenen Gesamt-Zeitintervall ergänzen:

| Temperatur-Zeit-Programm | |
|---|---|
| Temperatur (°C) | Zeitintervall |
| 90 ± 10 | 5 min ±5 min |
| 80 ± 10 | 10 min ± 10 min |
| 70 ± 10 | (30 bis 180 min) ± 30 |
| 40 ± 10 | (60 bis 180 min) ± 60 min |
| 25 ± 10 | 22 h ± 15 h |
| 4 ± 10 | 20 h ± 15 h |

**15.** Verfahren zur Herstellung von $\alpha$-Amylase-resistenten Polysacchariden, die einen nach der Methode von Englyst et al. ermittelten RS-Gehalt von mindestens 65 Gew.-% aufweisen, umfassend folgende Schritte:

a) Bereitstellung von wasserunlöslichem Poly-(1,4-$\alpha$-D-glukan) durch die Umsetzung von Saccharose mit einem Amylosucrase-Aktivität aufweisenden Enzym,

b) Herstellung einer Suspension oder Dispersion aus dem Poly-(1,4-$\alpha$-D-glukan) und Wasser;

c) Einfrieren der erhaltenen Suspension oder Dispersion;

d) Retrogradation;

e) Auftauen der nach Schritt (d) erhaltenen Masse; und

f) ggf. Trocknung oder Entwässerung der nach Schritt (e) erhaltenen Masse.

16. Verfahren nach Anspruch 15, **dadurch gekennzeichnet, dass** man die gemäß Schritt (e) aufgetaute Masse noch einmal oder mehrmals den Schritten (c) bis (e) unterwirft, bevor man endgültig auftaut, trocknet oder entwässert.

17. Verfahren nach einem der Ansprüche 15 oder 16, **dadurch gekennzeichnet, dass** man bei Schritt (c) gemäß Anspruch 15 die erhaltene Suspension oder Dispersion auf eine Temperatur im Bereich 0 °C bis -80 °C abkühlt.

18. Verfahren nach einem der Ansprüche 15 bis 17, **dadurch gekennzeichnet, dass** man bei Schritt (d) gemäß Anspruch 15 für ein Zeitintervall von 1 bis 72 h retrogradiert.

19. Verfahren nach einem der Ansprüche 10 bis 18, **dadurch gekennzeichnet, dass** die wasserunlöslichen Poly-(1,4-α-D-glukane) chemisch in an sich bekannter Weise modifiziert sind.

20. Verfahren nach einem der Ansprüche 10 bis 19, **dadurch gekennzeichnet, dass** die wasserunlöslichen Poly-(1,4-α-D-glukane) in 6-Position einen Verzweigungsgrad von höchstens 0,5 % aufweisen.

21. Verfahren nach einem der Ansprüche 10 bis 20, **dadurch gekennzeichnet, dass** die wasserunlöslichen Poly-(1,4-α-D-glukane) in 2- oder 3-Position einen Verzweigungsgrad von jeweils höchstens 0,5 % aufweisen.

22. Verfahren nach einem der Ansprüche 10 bis 21, **dadurch gekennzeichnet, dass** die wasserunlöslichen Poly-(1,4-α-D-glukane) ein Molekulargewicht von 0,75 x bis $10^2$ bis $10^7$ aufweisen.

23. Verfahren nach einem der Ansprüche 10 bis 22, **dadurch gekennzeichnet, dass** die wasserunlöslichen Poly-(1,4-α-D-glukane) weder entzweigt, noch hinsichtlich ihrer Kettenlänge (und damit hinsichtlich ihres Molekulargewichts) reduziert worden sind.

24. Verfahren nach einem der Ansprüche 10 bis 23, **dadurch gekennzeichnet, dass** man bei Schritt (e) gemäß Anspruch 10 oder bei Schritt (f) gemäß Anspruch 15 das retrogradierte Produkt durch Sprüh- oder Gefriertrocknung trocknet.

25. Verwendung eines α-Amylase-resistenten Polysaccharids gemäß einem der Ansprüche 1 bis 9 für Lebensmittel-Vorprodukte oder Lebensmittel.

26. Verwendung eines α-Amylase-resistenten Polysaccharids gemäß einem der Ansprüche 1 bis 9 als Lebensmitteladditiv oder Lebensmittelzusatzstoff.

27. Lebensmittel-Vorprodukt oder Lebensmittel, **gekennzeichnet durch** einen Gehalt an einem α-Amylase-resistenten Polysaccharid gemäß einem der Ansprüche 1 bis 9.

**Claims**

1. α-Amylase-resistant polysaccharides, <u>**characterised in that**</u> they exhibit a resistant starch(RS)-content of at least 65% by weight when tested by the method devised by Englyst et al and are obtainable by a procedure which consists of the following steps:

a) preparation of water-insoluble poly-(1,4-α-D-glucane) by reacting saccharose with an enzyme exhibiting amylosucrase activity,

b) manufacture of a suspension or dispersion from poly-(1,4-α-D-glucane) and water,

c) heating the suspension or dispersion in order to obtain a paste,

d) cooling the paste thus obtained and retrogradation of the paste at a temperature lower than that of the heated paste, and

e) if necessary, drying or de-watering the resulting product.

2. α-Amylase-resistant polysaccharides in accordance with claim 1, **characterised by** an RS-content of a minimum of 75%.

3. α-Amylase-resistant polysaccharides in accordance with claim 1 or 2, **characterised in that** the poly-(1,4-α-D-glucanes) are chemically modified by a known method.

4. α-Amylase-resistant polysaccharides in accordance with one of the foregoing claims, **characterised in that** they exhibit a degree of branching in the 6-position which is not greater than 0.5%.

5. α-Amylase-resistant polysaccharides in accordance with one of the foregoing claims, **characterised in that** they exhibit a degree of branching in each of the 2- and / or 3-positions which is not greater than 0.5%.

6. α-Amylase-resistant polysaccharides in accordance with one of the foregoing claims, **characterised in that** the poly-(1,4-α-D-glucanes) exhibit a molecular weight of between $0.75 \times 10^2$ and $10^7$ g / mol.

7. α-Amylase-resistant polysaccharides in accordance with one of the foregoing claims, **characterised in that** the poly-(1,4-α-D-glucanes) exhibit a molecular weight of between $10^3$ and $5 \times 10^5$ g / mol.

8. α-Amylase-resistant polysaccharides in accordance with one of the foregoing claims, **characterised in that** the poly-(1,4-α-D-glucanes) have not been de-branched - and especially not de-branched enzymatically - and also that the chain-lengths (and by association, the molecular weights) of the poly-(1,4-α-D-glucanes) have not been reduced.

9. α-Amylase-resistant polysaccharides, **characterised in that** they exhibit a RS-content of at least 65% by weight when tested by the method devised by Englyst et al and are obtainable by a procedure which embraces the following steps:

   a) preparation of water-insoluble poly-(1,4-α-D-glucane) by reacting saccharose with an enzyme exhibiting amylosucrase activity,
   b) manufacture of a suspension or dispersion from poly-(1,4-α-D-glucane) and water,
   c) freezing the resulting suspension or dispersion,
   d) retrogradation,
   e) thawing of the mass resulting from step d), and
   f) if necessary, drying or de-watering the mass resulting from step d).

10. Procedure for the manufacture of α-amylase-resistant polysaccharides which exhibit a RS-content of at least 65% by weight when tested by the method devised by Englyst et al and which consists of the following steps:

    a) preparation of water-insoluble poly-(1,4-α-D-glucane) by reacting saccharose with an enzyme exhibiting amylosucrase activity,
    b) manufacture of a suspension or dispersion from poly-(1,4-α-D-glucane) and water
    c) heating the resulting suspension or dispersion
    d) cooling the paste thus obtained and retrogradation of the paste at a temperature lower then that of the heated paste, and
    e) if necessary, drying or de-watering the resulting product.

11. Procedure in accordance with claim 10, **characterised in that** in the case of step b) in accordance with claim 10 a paste having a poly-saccharide content of at least 5 and up to 50 % by weight is produced.

12. Procedure in accordance with claim 10 or 11, **characterised in that** in the case of step c) in accordance with claim 10 the paste is heated to a temperature in the range between room temperature and 100°C.

13. Procedure in accordance with one of claims 10 to 12, **characterised in that** in the case of step d) in accordance with claim 10 retrogradation takes place

    (i) at a temperature in the range between 50 °C and the freezing point, and / or
    (ii) for a period of 1 to 72 hours.

14. Procedure in accordance with one of claims 10 to 13, **characterised in that** in the case of step (d) in accordance with claim 10 and conforming to a programme of temperature step changes

(i) over a temperature range of between 100 °C and 0 °C

(ii) step-wise over a total time interval of 8 to 36 hours in accordance with the following temperature-time programme and, if necessary, cooling and retrograding is carried out under the influence of shear forces, whereby the aggregate of the selected time intervals amounts to a pre-determined total time interval:

| Temperature-time programme | |
|---|---|
| Temperature (°C) | Time Interval |
| 90 +/- 10 | 5 min +/- 5 min |
| 80 +/- 10 | 10 min +/- 10 min |
| 70 +/- 10 | (30 to 180 min) +/- 30 |
| 40 +/- 10 | (60 to 180 min) +/- 60 min |
| 25 +/- 10 | 22 h +/- 15 h |
| 4 +/- 10 | 20 h +/- 15 h |

15. Procedure for the manufacture of $\alpha$-amylase-resistant polysaccharides which exhibit a RS-content of at least 65% by weight when tested by the method devised by Englyst et al which consists of the following steps:

   a) preparation of water-insoluble poly-(1,4-$\alpha$-D-glucane) by reacting saccharose with an enzyme exhibiting amylosucrase activity,
   b) manufacture of a suspension or dispersion from poly-(1,4-$\alpha$-D-glucane) and water,
   c) freezing the resulting suspension or dispersion,
   d) retrogradation,
   e) thawing of the mass resulting from step d), and
   f) if necessary, drying or de-watering the mass resulting from step e).

16. Procedure in accordance with claim 15, **characterised in that** the thawed mass resulting from step (e) is subjected once or several times more to the steps (c) to (e) before being finally thawed, dried or de-watered.

17. Procedure in accordance with one of the claims 15 or 16, **characterised in that** the suspension or dispersion obtained in step (c) in accordance with claim 15 is cooled to a temperature in the range between 0°C and -80 °C.

18. Procedure in accordance with one of the claims 15 to 17, **characterised in that** in step (d) in accordance with claim 15 retrogradation is carried out over a period of between 1 to 72 h.

19. Procedure in accordance with one of the claims 10 to 18, **characterised in that** the water-insoluble poly-(1,4-$\alpha$-D-glucanes) are modified chemically in a known manner.

20. Procedure in accordance with one of the claims 10 to 19, **characterised in that** the water-insoluble poly-(1,4-$\alpha$-D-glucanes) exhibit a degree of branching in the 6-position of a maximum of 0.5%.

21. Procedure in accordance with one of the claims 10 to 20, **characterised in that** the water-insoluble poly-(1,4-$\alpha$-D-glucanes) exhibit a degree of branching in each of the 2- or 3- positions which is not greater than 0.5%.

22. Procedure in accordance with one of the claims 10 to 21, **characterised in that**.the water-insoluble poly-(1,4-$\alpha$-D-glucanes) have a molecular weight of between $0.75 \times 10^2$ and $10^7$.

23. Procedure in accordance with one of claims 10 to 22, **characterised in that**. the poly-(1,4-$\alpha$-D-glucanes) have neither been de-branched nor have had their chain-lengths (and by association, the molecular weights) reduced.

24. Procedure in accordance with one of the claims 10 to 23, **characterised in that** in step (e) in accordance with claim 10 or in step (f) in accordance with claim 15 the retrograded product is spray-dried or freeze-dried.

25. Use of an $\alpha$-amylase-resistant polysaccharide in accordance with one of the claims 1 to 9 for foodstuff precursors or foodstuffs.

**26.** Use of an α-amylase-resistant polysaccharide in accordance with one of the claims I to 9 as a foodstuff additive or as a foodstuff ingredient

**27.** Foodstuff-precursor or foodstuff(s) **characterised by** a content of an α-amylase-resistant polysaccharide in accordance with one of the claims 1 to 9.

**Revendications**

**1.** Polysaccharides résistants à l'α-amylase, **caractérisés en ce qu'**ils présentent une teneur en RS d'au moins 65 % en masse, déterminée selon la méthode d'Englyst et al., que l'on peut obtenir selon un procédé comprenant les étapes suivantes :

a) préparation de poly-(1,4-α-D-glucane) insoluble dans l'eau par la réaction du saccharose sur une enzyme présentant une activité d'amylosucrase,
b) préparation d'une suspension ou dispersion de poly-(1,4-α-D-glucane) et d'eau,
c) chauffage de la suspension ou dispersion afin d'obtenir une colle d'amidon ;
d) refroidissement de la colle d'amidon obtenue et rétrogradation de la colle d'amidon à une température réduite par rapport à la température de la colle d'amidon chauffée ; et
e) éventuellement séchage ou déshydratation du produit obtenu.

**2.** Polysaccharides résistants à l'α-amylase selon la revendication 1, **caractérisés par** une teneur en RS d'au moins 75 %.

**3.** Polysaccharides résistants à l'α-amylase selon la revendication 1 ou 2, **caractérisés en ce que** les poly-(1,4-α-D-glucanes) sont modifiés par voie chimique de façon connue en soi.

**4.** Polysaccharides résistants à l'α-amylase selon l'une des revendications précédentes, **caractérisés en ce qu'**ils présentent en position 6 un degré de ramification d'au maximum 0,5 %.

**5.** Polysaccharides résistants à l'α-amylase selon l'une des revendications précédentes, **caractérisés en ce qu'**ils présentent en positions 2 et/ou 3 un degré de ramification d'au maximum 0,5 %.

**6.** Polysaccharides résistants à l'α-amylase selon l'une des revendications précédentes, **caractérisés en ce que** les poly-(1,4-α-D-glucanes) présentent une masse moléculaire de $0,75 \times 10^2$ à $10^7$ g/mole.

**7.** Polysaccharides résistants à l'α-amylase selon l'une des revendications précédentes, **caractérisés en ce que** les poly-(1,4-α-D-glucanes) présentent une masse moléculaire de $10^3$ à $5 \times 10^5$ g/mole.

**8.** Polysaccharides résistants à l'α-amylase selon l'une des revendications précédentes, **caractérisés en ce que** les poly-(1,4-α-D-glucanes) ne sont ni débranchés, en particulier débranchés par voie enzymatique, ni la longueur de leur chaîne n'est réduite (et de ce fait, concernant leur masse moléculaire).

**9.** Polysaccharides résistants à l'α-amylase, **caractérisés en ce qu'**ils présentent une teneur en RS d'au moins 65 % en masse, déterminée selon la méthode d'Englyst et al., que l'on peut obtenir selon un procédé comprenant les étapes suivantes :

a) préparation de poly-(1,4-α-D-glucane) insoluble dans l'eau par réaction de saccharose sur une enzyme présentant une activité d'amylosucrase,
b) préparation d'une suspension ou dispersion de poly-(1,4-α-D-glucane) et d'eau,
c) congélation de la suspension ou dispersion obtenues ;
d) rétrogradation ;
e) décongélation de la matière obtenue selon l'étape d) ; et
f) éventuellement séchage ou déshydratation de la matière obtenue selon l'étape d).

**10.** Procédé pour la préparation de polysaccharides résistants à l'α-amylase, qui présentent une teneur en RS d'au moins 65 % en masse, déterminée selon la méthode d'Englyst et al., comprenant les étapes suivantes :

a) préparation de poly-(1,4-$\alpha$-D-glucane) insoluble dans l'eau par la réaction de saccharose sur une enzyme présentant une activité d'amylosucrase,

b) préparation d'une suspension ou dispersion de poly-(1,4-$\alpha$-D-glucanes) insolubles dans l'eau et d'eau,

c) chauffage de la suspension ou dispersion ;

d) refroidissement de la colle d'amidon obtenue et rétrogradation de la colle d'amidon à une température réduite par rapport à la température de la colle chauffée ; et

e) éventuellement séchage ou déshydratation du produit obtenu.

**11.** Procédé selon la revendication 10, **caractérisé** en qu'on prépare à l'étape (b) selon la revendication 10 une colle d'amidon présentant une teneur en polysaccharide d'au moins 5 et à 50 % en masse.

**12.** Procédé selon la revendication 10 ou 11, **caractérisé** en qu'on chauffe la colle d'amidon à l'étape (c) selon la revendication 10 à une température dans le domaine allant de la température ambiante à 100°C.

**13.** Procédé selon l'une des revendications 10 à 12, **caractérisé en ce qu'**on rétrograde à l'étape (d) selon la revendication 10

(i) à une température dans le domaine de 50°C au point de congélation, et/ou

(ii) pendant une durée de 1 à 72 heures.

**14.** Procédé selon l'une des revendications 10 à 13, **caractérisé en ce que**, à l'étape (d) selon la revendication 10, selon un régime de températures progressif

(i) dans un domaine de températures de 100 à 0°C

(ii) pendant une durée totale de 8 à 36 heures, on refroidit et rétrograde progressivement et éventuellement sous l'effet de forces de cisaillement selon le programme température-durée progressif suivant, les intervalles de temps choisis se complètent à donner un intervalle de temps total indiqué ci-dessus :

| Programme température-durée | |
| --- | --- |
| Température (°C) | Intervalle de temps |
| 90 $\pm$ 10 | 5 min $\pm$ 5 min |
| 80 $\pm$ 10 | 10 min $\pm$ 10 min |
| 70 + 10 | (30 à 180 min) $\pm$ 30 |
| 40 $\pm$ 10 | (60 à 180 min) $\pm$ 60 min |
| 25 $\pm$ 10 | 22 h $\pm$ 15 h |
| 4 $\pm$ 10 | 20 h $\pm$ 15 h |

**15.** Procédé pour la préparation de polysaccharides résistants à l'$\alpha$-amylase, qui présentent une teneur en RS d'au moins 65 % en masse, déterminée selon la méthode d'Englyst et al., comprenant les étapes suivantes :

a) préparation de poly-(1,4-$\alpha$-D-glucane) insoluble dans l'eau par réaction de saccharose sur une enzyme présentant une activité d'amylosucrase,

b) préparation d'une suspension ou dispersion de poly-(1,4-$\alpha$-D-glucane) et d'eau,

c) congélation de la suspension ou dispersion obtenues ;

d) rétrogradation ;

e) décongélation de la matière obtenue selon l'étape (d) ; et

f) éventuellement séchage ou déshydratation de la matière obtenue selon l'étape (e).

**16.** Procédé selon la revendication 15, **caractérisé en ce qu'**on soumet la matière décongelée selon l'étape (e) encore une fois ou plusieurs fois aux étapes (c) à (e), avant qu'elle soit complètement décongelée, séchée ou déshydratée.

**17.** Procédé selon l'une des revendications 15 ou 16, **caractérisé en ce qu'**on refroidit la suspension ou dispersion obtenues à l'étape (c) selon la revendication 15 à une température dans le domaine de 0°C à -80°C.

**18.** Procédé selon l'une des revendications 15 à 17, **caractérisé en ce qu'**on rétrograde à l'étape (d) selon la revendication 15 pendant un intervalle de temps de 1 à 72 heures.

19. Procédé selon l'une des revendications 10 à 18, **caractérisé en ce que** les poly-(1,4-α-D-glucanes) insolubles dans l'eau sont modifiés par voie chimique de façon connue en soi.

20. Procédé selon l'une des revendications 10 à 19, **caractérisé en ce que** les poly-(1,4-α-D-glucanes) insolubles dans l'eau présentent en position 6 un degré de ramification d'au maximum 0,5 %.

21. Procédé selon l'une des revendications 10 à 20, **caractérisé en ce que** les poly-(1,4-α-D-glucanes) insolubles dans l'eau présentent en positions 2 ou 3 un degré de ramification respectivement d'au maximum 0,5 %.

22. Procédé selon l'une des revendications 10 à 21, **caractérisé en ce que** les poly-(1,4-α-D-glucanes) insolubles dans l'eau présentent une masse moléculaire de 0,75x à $10^2$ à $10^7$.

23. Procédé selon l'une des revendications 10 à 22, **caractérisé en ce que** les poly-(1,4-α-D-glucanes) insolubles dans l'eau ne sont ni débranchés, ni réduits en ce qui concerne la longueur de leur chaîne (et de ce fait, en ce qui concerne leur masse moléculaire).

24. Procédé selon l'une des revendications 10 à 23, **caractérisé en ce que** le produit rétrogradé à l'étape (e) selon la revendication 10 ou à l'étape (f) selon la revendication 15 est séché par pulvérisation ou lyophilisation.

25. Utilisation d'un polysaccharide résistant à l'α-amylase selon l'une des revendications 1 à 9 pour des précurseurs de produits alimentaires ou pour des produits alimentaires.

26. Utilisation d'un polysaccharide résistant à l'α-amylase selon l'une des revendications 1 à 9 en tant qu'additif de produits alimentaires ou matière d'addition de produits alimentaires.

27. Précurseur de produits alimentaires ou produit alimentaire, **caractérisé par** une teneur en un polysaccharide résistant à l'α-amylase selon une des revendications 1 à 9.

Fig. 1   GPC - Chromatogramm des Poly-(1,4-α-D-glukans)